# EUROPEAN PATENT APPLICATION

(11) **EP 1 882 940 A1**
(43) Date of publication of application: **30.01.2008**
(21) Application number: 06015677.5
(22) Date of filing: 27.07.2006
(51) Int. Cl.: G01N 33/543, G01N 33/558

(54) **Chitosan as a colour-fixing agent**

(71) Applicant: The Jordanian Pharmaceutical Manufacturing Co., 11710 Naor (JO)
(72) Inventor: Adnan, Badwan, Dr., 11185 Amman (JO); Murshed, Abedel-Qader Mohammed, 11118 Amman (JO)
(74) Representative: Winkler, Andreas Fritz Ernst

(57) **Abstract**

The present invention refers to an indication means comprising an indicator, preferably a humidity colour indicator, and chitosan. The invention further refers to a lateral-flow test device comprising said indication means, and to a use of said test device for diagnosis, prognosis and/or observation of a disease. The invention also refers to a use of chitosan for the preparation of an indication means and/or a lateral-flow test device.

## Description

The present invention refers to an indication means comprising an indicator, preferably a humidity colour indicator, and chitosan. The invention further refers to a lateral-flow test device comprising said indication means, and to a use of said test device for diagnosis, prognosis and/or observation of a disease. The invention also refers to a use of chitosan for the preparation of an indication means and/or a lateral-flow test device.

### Background of the Invention

Inorganic compounds changing colour upon contact with water molecules are used in qualitative detection of humidity for a long time. Such indicator material has found application also for diagnostic purposes where changes in relative humidity are of critical importance. For example, it allows visual inspection of the condition of a sealed package by readily detecting and indicating any change in relative humidity.

Examples of inorganic compounds serving as humidity indicators are cobalt chloride, copper chloride or lead iodide compounds. Cobalt (II) chloride forms hydrates of different colours ranging between blue-violet (CoCl₂· H₂O) and pink (CoCl₂· 6H₂O). Copper chloride changes colour from brown to azure in the presence of water molecules. Solutions of KPbI₃ have been used to impregnate filter paper for detecting traces of water since the complex is split in the presence of water leading to coloured PbI₂.

In general, the occurrence of colour change of a humidity colour indicator depends on the ratio of water and indicator molecules. Thus, referring to a constant amount of water molecules (and assuming that other influences such as ambient conditions are negligible), colour changes the later the greater the excess of indicator molecules is.

Humidity colour indicators can be employed in lateral-flow test devices not only for visualisation of a change in relative humidity but also for detecting and indicating the arrival of an ascending aqueous sample at the test zone. Thus, colour change can be used to precisely determine the time when the sample to be tested gets into contact with the detection reagent whereupon the detection reaction is initiated. Depending on the configuration of the device, colour change can also be used to indicate the time when the test result is ready for read. However, once appeared upon contact of indicator and sample, the colour faints over time due to a diffusion of the indicator molecules. This process is difficult to control and must not correlate with the time period during which the result can be accurately be read. Therefore, it would be advantageous to the user to know since when the result can be read but also since when the result should not be read any more.

Consequently, there is a need of a lateral-flow test device providing information about the time period during which a test result can be read accurately.

### Summary of the Invention

The object of the present invention is solved by an indication means comprising an indicator and chitosan.

In one embodiment, the indication means serves time indication, i.e. is a time indication means.

In one embodiment, the indication means further comprises a substrate. Preferably, the substrate comprises cellulose and/or nitrocellulose. The substrate can be, for example, a cellulose pad.

In one embodiment, the indication is based on optical determination, i.e. the indicator material is an optical indicator. Preferably, the optical indicator works by optical determination of a colour change, appearance or disappearance, i.e. is a colour indicator material. More preferably, the colour indicator material is selected from a humidity and a pH indicators. Even more preferably, the humidity colour indicator is selected from cobalt chloride, copper chloride and lead iodide compounds. Most preferably, the humidity colour indicator is cobalt (II) chloride.

In principle, any material can be used as an indicator that visually changes colour once the sample to be tested gets into contact therewith. In addition to the colour indicators mentioned above, other indicators are considered, e.g. adsorption, redox and metallochrome indicators, protein and carbon hydrate staining agents, and colour precipitates resulting from enzymatic reactions.

In one embodiment, the ratio of chitosan and humidity colour indicator is selected from 1:7.5, 1:12.5, and 1:16.25.

In one embodiment, the ratio of chitosan and humidity colour indicator is selected according to the desired time of colour change and colour disappearance.

The object of the present invention is further solved by a lateral-flow test device comprising at least one indication means according to the present invention.

In one embodiment, the test device further comprises at least one indication means for detection and quantification of an analyte selected from hCG antigen, *Helicobacter pylori* antibody and HIV antibody. However, also other analytes such as penicillins, tumour markers, hepatitis B surface antigen or luteinizing hormone is take into account.

In a preferred embodiment, the test device comprises an indication means for detection and quantification of hCG antigen, wherein the time indication means comprises chitosan and a humidity colour indicator material at a ratio of 1:7.5.

In another preferred embodiment, the test device comprises an indication means for detection and quantification of *Helicobacter pylori* antibody, wherein the time indication means comprises chitosan and humidity colour indicator material at a ratio of 1:12.5.

In yet another preferred embodiment, the test device comprises an indication means for detection and quantification of HIV antibody, wherein the time indication means comprises chitosan and humidity colour indicator material at a ratio of 1:16.25.

The object of the present invention is further solved by a use of the test device for diagnosis, prognosis and/or monitoring of a disease.

In one embodiment, the disease is selected form *Helicobacter pylori,* HIV, HBV, HCV infection and tuberculosis.

The object of the present invention is further solved by a use of chitosan for fixing a humidity colour indicator material.

In one embodiment, the humidity colour indicator material is selected from cobalt chloride, copper chloride and lead iodide compounds. Preferably, the humidity colour indicator is cobalt (II) chloride.

The object of the present invention is further solved by a use of chitosan for the preparation of an indication means according to the present invention and/or for the preparation of a lateral-flow test device according to the present invention.

The object of the present invention is further solved by a procedure of manufacture of a time indication means according to the present invention comprising an application of chitosan onto a substrate.

In one embodiment, the substrate comprises cellulose and/or nitrocellulose.

In one embodiment, the application comprises a spraying of and/or impregnating with an aqueous solution of chitosan.

In one embodiment, the application of chitosan is prior to, along with and/or after the application of a humidity colour indicator.

The present invention makes chitosan available for use as a colour-fixing agent. The mechanism is based on the property of chitosan to form cross-links with e.g. cellulose or nitrocellulose. The resulting structure allows for entrapping colour indicator molecules. The ratio of chitosan and colour indicator determines the time period after which the colour indication molecules are washed out, e.g. by diffusion in an aqueous sample to be tested. Thus, the addition or admixture of chitosan to colour indicator material allows for a modulation of the time period after which a colour change, previously occurred upon contact with the sample, can not be ascertained any longer.

The colour-fixing property of chitosan is particularly useful in time indication means. For realizing the present invention, a mixture of indicator and chitosan can be applied onto a cellulose or nitrocellulose substrate, e.g. by spraying an appropriate solution on cards or pads in form of spots, by immersion cards or pads in this solution or by otherwise impregnating cards or pads. Chitosan can be applied in admixture with the indicator or in a separate step.

Applying different amounts of humidity indication material and different concentrations of its solution, respectively, the sensitivity of humidity indication may be varied. Furthermore, the kind of humidity indication material may be selected according to the preferred sensitivity. Also the thickness and location of the support may be selected with regard to the preferred sensitivity.

### Detailed Description of the Invention

### Brief Description of the Figures

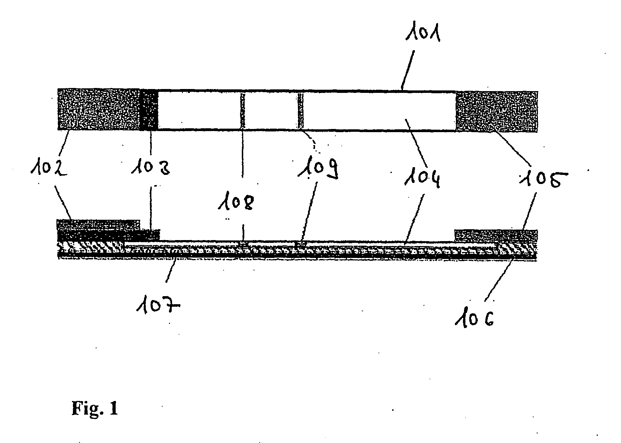

Figure 1 shows top and side views of a typical rapid-flow immunochromatographic test device in the form of a test strip 101 including a sample pad 102, a conjugate pad 103, a membrane 104, e.g. a nitrocellulose membrane, an absorbent pad 105, an adhesive 106, a supporting backing 107, e.g. made of plastic, a capture (test) zone 108, and a control (negative) zone 109.

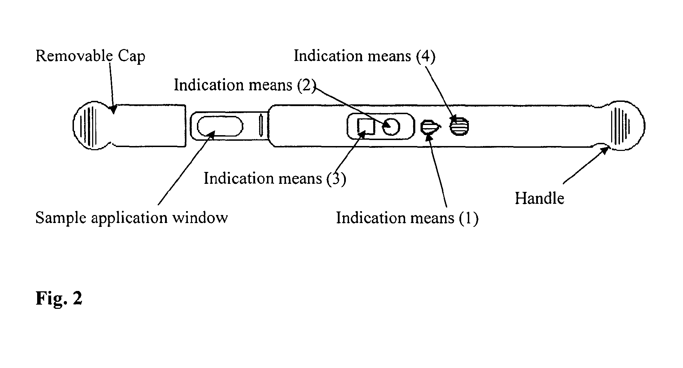

Figure 2 shows a preferred embodiment of a lateral-flow test device of the present invention comprising indication means, namely:
   an indication means (1) realized by a result reading time indication hole;
   an indication means (2) realized by a control line region;
   an indication means (3) realized by a test line region;
   an indication means (4) realized by a humidity indication hole.
Figure 3 demonstrates the operation of a lateral-flow test device as shown Figure 2 with indication means (1) and (4) comprising cobalt chloride as the humidity indicator.
Figure 4 demonstrates the operation of a lateral flow test device as shown Figure 2 with indication means (1) and (4) comprising copper chloride as the humidity indicator.

### EXAMPLES

### EXAMPLE 1: A lateral-flow test device of the present invention using cobalt chloride

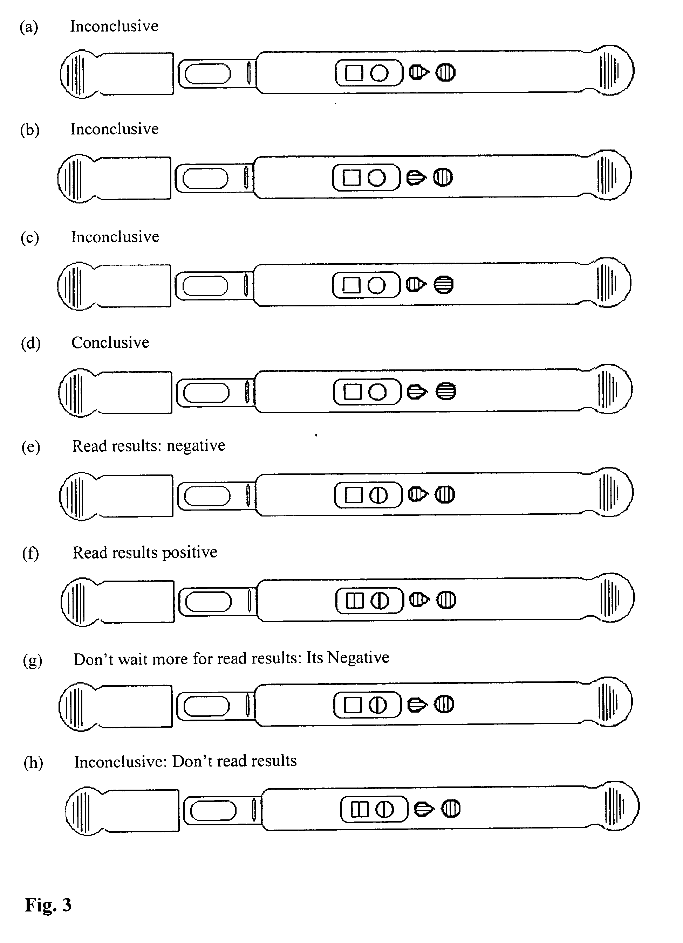

It is referred to Figure 3. Both indication means (4) (= humidity indication hole) and indication means (1) (= results reading time indication hole) comprise cobalt chloride as the indicator material, optionally with other additives to meet the intensified color change requirements. Indication means (1) together with indication means (2) (= control line region) and (3) (= test line region) are constructed such that they get in contact with a sample applied to the sample application window. In contrast, means (4) is not intended to physically contact the sample, e.g. by fluid communication. As a consequence, indication means (4) and indication means (1) are capable of indicating high relative atmospheric humidity, e.g. more than 40%, whereas only indication means (1) is capable of indicating that the sample has passed indication means (3) and (2), so the reaction is completed and thus the test result is ready for read.

The information provided by a cobalt chloride test device as described is drawn up below (see also Figure 3.

| | | |
|---|---|---|
| (a) | Indication means (1): | pink |
| | Indication means (2): | blank |
| | Indication means (3): | blank |
| | Indication means (4): | pink |
| | Result: | inconclusive |
| | Comment: | The test must be repeated with new device. |
| | | |
| (b) | Indication means (1): | blue |
| | Indication means (2): | blank |
| | Indication means (3): | blank |
| | Indication means (4): | pink |
| | Result: | inconclusive |
| | Comment: | The test must be repeated with new device. |
| | | |
| (c) | Indication means (1): | pink |
| | Indication means (2): | blank |
| | Indication means (3): | blank |
| | Indication means (4): | blue |
| | Result: | inconclusive |
| | Comment: | The test must be repeated with new device. |
| | | |
| (d) | Indication means (1): | blue |
| | Indication means (2): | blank |
| | Indication means (3): | blank |
| | Indication means (4): | blue |
| | Result: | conclusive |
| | Comment: | The test device can be safely used. |
| | | |
| (e) | Indication means (1): | pink |
| | Indication means (2): | pink/purple band |
| | Indication means (3): | blank |
| | Indication means (4): | pink |
| | Result: | negative |
| | | |
| (f) | Indication means (1): | pink |
| | Indication means (2): | pink/purple band |
| | Indication means (3): | pink/purple band |
| | Indication means (4): | pink |
| | Result: | positive |
| (g) | Indication means (1): | blank |
| | Indication means (2): | pink/purple band |
| | Indication means (3): | blank |
| | Indication means (4): | pink |
| | Result: | negative |
| | Comment: | Don't wait more for reading results. |
| | | |
| (h) | Indication means (1): | blank |
| | Indication means (2): | pink/purple band |
| | Indication means (3): | pink/purple band |
| | Indication means (4): | pink |
| | Result: | inconclusive |
| | Comment: | Don't read results. |

### EXAMPLE 2: Operation of a test device of the present invention using copper chloride.

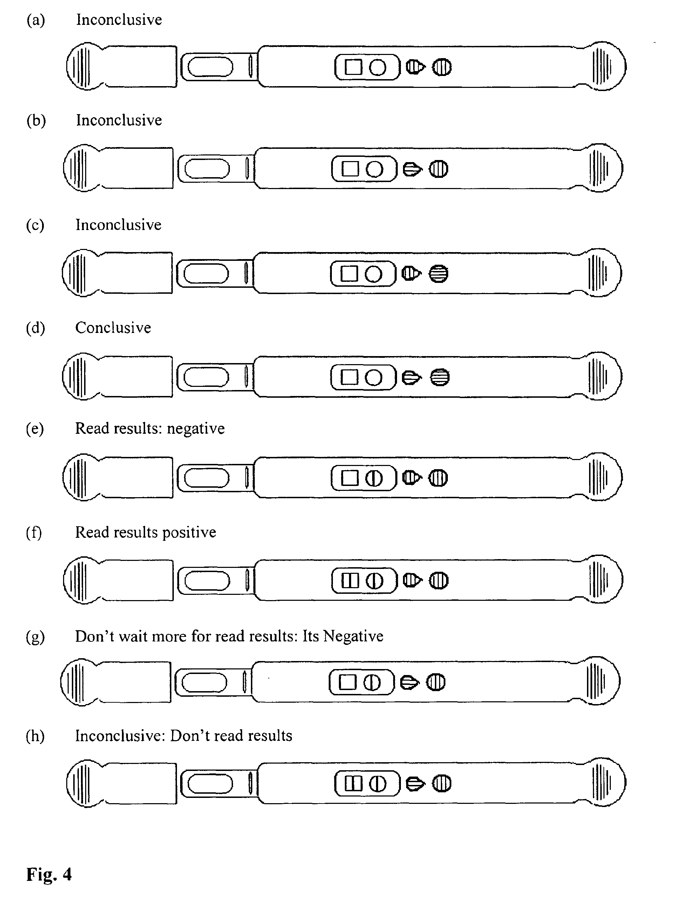

It is referred to Figure 4. In contrast to the test device of Example 1, this test device comprises copper chloride instead of cobalt chloride as the humidity indicator material.

The information provided by a copper chloride test device as described is drawn up below (see also Figure 4).

| | | |
|---|---|---|
| (a) | Indication means (1): | azure |
| | Indication means (2): | blank |
| | Indication means (3): | blank |
| | Indication means (4): | azure |
| | Result: | inconclusive |
| | Comment: | The test must be repeated with new device. |
| | | |
| (b) | Indication means (1): | brown |
| | Indication means (2): | blank |
| | Indication means (3): | blank |
| | Indication means (4): | azure |
| | Result: | inconclusive |
| | Comment: | The test must be repeated with new device. |
| | | |
| (c) | Indication means (1): | azure |
| | Indication means (2): | blank |
| | Indication means (3): | blank |
| | Indication means (4): | brown |
| | Result: | inconclusive |
| | Comment: | The test must be repeated with new device. |
| | | |
| (d) | Indication means (1): | brown |
| | Indication means (2): | blank |
| | Indication means (3): | blank |
| | Indication means (4): | Brown |
| | Result: | conclusive |
| | Comment: | The test device can be safely used. |
| | | |
| (e) | Indication means (1): | azure |
| | Indication means (2): | pink/purple band |
| | Indication means (3): | blank |
| | Indication means (4): | azure |
| | Result: | negative |
| | | |
| (f) | Indication means (1): | azure |
| | Indication means (2): | pink/purple band |
| | Indication means (3): | pink/purple band |
| | Indication means (4): | azure |
| | Result: | positive |
| | | |
| (g) | Indication means (1): | blank |
| | Indication means (2): | pink/purple band |
| | Indication means (3): | blank |
| | Indication means (4): | azure |
| | Result: | negative |
| | Comment: | Don't wait more for reading results. |
| | | |
| (h) | Indication means (1): | blank |
| | Indication means (2): | pink/purple, band |
| | Indication means (3): | pink/purple band |
| | Indication means (4): | Azure |
| | Result: | Inconclusive |
| | Comment: | Don't read results. |

### EXAMPLE 3: hCG antigen detection

Colour changes within 1-2 minutes (ready to read results), and disappears within 15-20 minutes (don't read results) by considering the following parameters:
(a) High flow rate system components by using low capillary rise of nitrocellulose membrane and 18 drops of urine sample.
(b) High concentration of printed antibodies (using the concentration of 1.0mg/ml of antibody printing solution).
(c) Spray 6 microliter of indication mixture per centimetre of the substrate.
(d) The indication mixture is composed of one part of chitosan (2.0 % water-soluble chitosan in water) and one part of cobalt chloride solution (15.0% cobalt chloride hexahydrate in water). Thus, the ratio of chitosan and cobalt chloride is 2:15, i.e. 1:7.5.

### EXAMPLE 4: H. pylori antibody detection

Colour changes within 10 minutes (ready to read results), and disappears within 30 minutes (don't read results) by considering the following parameters:
(a) Slow flow rate system components by using a high capillary rise nitrocellulose membrane and 14 drops of assay buffer.
(b) Spray 6 microliter of indication mixture per centimetre of the substrate.
(c) The indication mixture is composed of two parts of chitosan (2.0% water-soluble chitosan in water) and one part of cobalt chloride solution (50.0% cobalt chloride hexahydrate in water). Thus, the ratio of chitosan and cobalt chloride is 4:50, i.e. 1:12.5.

### EXAMPLE 5: HIV antibody detection

Colour changes within 15 minutes (ready to read results), and disappears within 30 minutes (don't read result) by considering the following parameters:
(a) Very slow flow rate system components by using a higher capillary rise nitrocellulose membrane and 12 drops of assay buffer.
(b) Spray 6 microliter of indication mixture per centimetre of the substrate.
(c) The indication mixture is composed of two parts of chitosan (2.0% water-soluble chitosan in water) and one part of cobalt chloride solution (65.0% cobalt chloride hexahydrate in water). Thus, the ratio of chitosan and cobalt chloride is 4:65, i.e. 1:16.25.

## Claims

1. A time indication means comprising an indicator and chitosan.

2. The time indication means according to claim 1, further comprising a substrate, said substrate preferably comprising cellulose and/or nitrocellulose.

3. The time indication means according to claim 1 or 2, wherein the indicator is a colour indicator, preferably a humidity colour indicator or a pH colour indicator.

4. The time indication means according to claim 3, wherein the humidity colour indicator is selected from cobalt chloride, copper chloride and lead iodide compounds.

5. The time indication means according to claim 3 or 4, wherein the humidity colour indicator is cobalt (II) chloride.

6. The time indication means according to any of the preceding claims, wherein the ratio of chitosan and humidity colour indicator is selected from 1:7.5, 1:12.5, and 1:16.5.

7. The time indication means according to any of claims 1 to 5, wherein the ratio of chitosan and humidity colour indicator is selected according to the desired time of colour change and colour disappearance.

8. A lateral-flow test device comprising at least one time indication means according to any of claims 1 to 7.

9. The test device according to claim 8, further comprising at least one indication means for detection and quantification of an analyte selected from hCG antigen, *Helicobacter pylori* antibody and HIV antibody.

10. The test device according to claim 8 or 9, comprising an indication means for detection and quantification of hCG antigen, wherein the time indication means comprises chitosan and a humidity colour indicator at the ratio of 1:7.5.

11. The test device according to claim 8 or 9, comprising an indication means for detection and quantification of *Helicobacter pylori* antibody, wherein the time indication means comprises chitosan and humidity colour indicator at the ratio of 1:12.5.

12. The test device according to claim 8 or 9, comprising an indication means for detection and quantification of HIV antibody, wherein the time indication means comprises chitosan and humidity colour indicator at the ratio of 1:16.5.

13. A use of the test device according to any of claims 7 to 12 for diagnosis, prognosis and/or monitoring of a disease.

14. The use according to claim 13, wherein the disease is selected form *Helicobacter pylori,* HIV , HBV, HCV infection or tuberculosis.

15. A use of chitosan for fixation of a humidity colour indicator.

16. The use according to claim 15, wherein the humidity colour indicator is selected from cobalt chloride, copper chloride and lead iodide compounds.

17. The use according to claim 16, wherein the humidity colour indicator is cobalt (II) chloride.

18. The use according to any of claims 15 to 17, wherein the ratio of chitosan and humidity colour indicator is selected from 1:7.5, 1:12.5, and 1:16.5.

19. A use of chitosan for the preparation of an indication means according to any of claims 1 to 5 and/or for the preparation of a lateral-flow test device according to any of claims 8 to 12.

20. A procedure of manufacture of a time indication means according to any of claims 1 to 6, comprising an application of chitosan onto a substrate.

21. The procedure according to claim 20, wherein the substrate comprises cellulose and/or nitrocellulose.

22. The procedure according to claim 20 or 21, wherein the application comprises a spraying of or impregnating with an aqueous solution of chitosan.

23. The procedure according to any of claims 20 to 22, wherein the application of chitosan is prior to, along with and/or after the application of a humidity colour indicator.
